# EUROPEAN PATENT SPECIFICATION

(11) **EP 2 640 525 B1**
(45) Date of publication and mention of the grant of the patent: **03.01.2018**
(21) Application number: 11785006.5
(22) Date of filing: 17.11.2011
(51) Int. Cl.: B05B 7/26, B05B 11/00

(54) **SPRAY APPARATUS AND METHOD FOR SPRAYING FRAGRANCE AND WATER**
SPRÜHGERÄT UND METHODE ZUM VERSPRÜHEN VON PARFÜM UND WASSER
DISPOSITIF DE PULVÉRISATION ET MÉTHODE DE PULVÉRISATION DE PARFUM ET D'EAU

(30) Priority: 17.11.2010 GB 201019427
(43) Date of publication of application: 25.09.2013
(73) Proprietor: Givaudan SA, 1214 Vernier (CH)
(72) Inventor: QUELLET, Christian, CH-2502 Biel (CH)
(74) Representative: Global Patents
(86) International application number: PCT/EP2011/070334
(87) International publication number: WO 2012/066068

(56) References cited:
- EP-A1- 1 421 997
- EP-A2- 0 479 451
- WO-A1-2010/052388
- FR-A- 1 408 299
- US-A1- 2008 223 953
- US-B1- 6 189 810

## Description

This disclosure relates to fragrance and to its dissemination into an atmosphere.

Fragrance in an atmosphere or sprayed on to a substrate (including skin) is often desired for a number of aesthetic and practical reasons, for example, to freshen air, to provide an attractive personal fragrance, or a particular mood or ambience in a room. It is ineffective (and quite wasteful) to spray neat fine fragrance into an atmosphere or on to a surface, as it may be perceived as too intense, and ways have been sought to use less fragrance and, at the same time, to make it more effective.

One such method well known to the art is to dissolve or disperse the fragrance in an organic solvent or a blend of such solvents. The most commonly-used solvent has been ethanol, one of the more celebrated products of this type being eau de Cologne. Although these have been effective, there is a desire to move away from these formulations on cost and environmental grounds.

The most preferable solution is water, but the incorporation into water of generally hydrophobic fragrance ingredients has meant the need for the preparation of emulsions, using emulsifiers, sometimes in substantial quantities. So far, these have not proved as effective as the solvent-based systems. They often have the additional disadvantage of a limited shelf life, because of the emulsion nature. In particular, emulsifiers can leave undesirable residues on substrates, especially skin, and produce thereby sticky or simply unpleasant residues. Furthermore, emulsifiers can interact with water and slow down its evaporation speed, leading to an undesirable moist feeling. Emulsifiers can also retain certain perfume ingredients, leading to loss of perfume performance and perfume profile distortion, and produce undesired foam. Finally, emulsions have the additional disadvantage of a limited shelf life, because of their thermodynamically unstable nature.

The use of large amounts of water in perfume compositions may also induce undesired chemical reactions, such as hydrolysis and oxidation, affecting the integrity of some perfume ingredients, especially natural ingredients, and the overall olfactive characteristics of the perfume. One alternative method is described in WO 2010/052388, but again this involves creating an emulsion. Document WO2010/052388 discloses the features or method steps, respectively, of the preamble of claims 1 and 6. It has now been found that these problems in the art can be at least substantially overcome by means of a particular method. There is therefore provided a method of providing a fine mist of sprayed fragrance, comprising the provision of separate reservoirs of water and fragrance and the conveying of the liquids through separate conduits to at least one spray nozzle, the liquids being combined immediately after spraying from the at least one nozzle.

There is additionally provided a spray device, comprising separate reservoirs of fragrance and water, each reservoir having a conduit leading therefrom to at least one spray nozzle, the at least one spray nozzle and the conduits being configured such that the two liquids are combined immediately after spraying from the at least one nozzle.

By "water" is meant primarily tap, distilled or deionized water, which is substantially free of any emulsifier. However, in a specific embodiment of this disclosure, the water may also contain up to 2 wt%, preferably less than 1 wt% and most preferably less than 0.5% of additives, such as viscosity controlling agents, surface active agents, preservatives, dyestuffs and colouring matters, and the like, which are added in order to improve the flow properties, the aerosol droplet size and droplet size distribution, the aspect and the stability of water against bio-contaminations.
The device may be made from any suitable material and of any desired capacity. Appropriate proportions of both reservoirs and conduits can easily be ascertained by the skilled person with only simple experimentation.
The spray nozzle for use in the device hereinabove defined may be any suitable spray nozzle capable of producing a spray of fine droplets, or aerosol. The formation of the aerosol may be by any convenient means, such as simple pressure (from, for example, a manual pump or by means of pressurised gas) or with the use of piezoelectric, ionisation, cavitation or sound (including ultrasound) devices. Combinations of two or more of such devices may also be used.

By "at least one spray nozzle" is meant one nozzle or a plurality of nozzles, in the latter case typically two. In the case of a plurality of nozzles, separate sprays are produced and merged as they spray.

In the case of a single nozzle, the liquids may be combined immediately prior to spraying. Alternatively, one may be sprayed and the other emitted from a conduit whose end is located in the direct path of the spray, such that the liquid emitted from the conduit is atomised by the spray and transported. In this case, it is typically the water that is sprayed and the fragrance emitted from the conduit, but this may be reversed.

In a single nozzle in which a combined spray is produced, the two conduits may feed into a mixing chamber adjacent to the spray nozzle, and may be sprayed from it. The mixing chamber may additionally contain a static mixer. The two conduits may have separate entrances into the mixing chamber, but in some cases it can be difficult to ensure that, if there is liquid in the chamber after spraying is concluded, the mixed liquids do not flow into the reservoirs, contaminating them. A particular embodiment avoids this problem. In this embodiment, the conduits are concentric for the final parts of their lengths to the chamber. This enables undesirable flow-back to be prevented by relatively simple means, such as a valve in either or both conduits. An example is a spring-loaded ball valve at the bottom of the chamber. Such devices are already well known, are cheaply and readily available and are widely used in simple pumping devices of all kinds. There are of course alternative ways of achieving the same end result, and the skilled person will be able to provide such alternatives.

The surprising feature of this single nozzle-mixing chamber device is that incompatible liquids may be combined to produce an effective fragrant spray, in that the fragrance retains its integrity, even though it may be a complex blend of a number of ingredients of varying hydrophobicity. The precise nature of the sprayed aerosol is not known, but it is believed that fragrance is adsorbed on to the surface of water droplets formed by the spraying action, and are thus carried into the atmosphere, or on to a surface.

In a two-nozzle device, two sprays are produced, one water spray, the other fragrance spray, and the two sprays are combined to give a single fragrant spray. This requires that the nozzles be positioned to give rise to such a combined spray. This will involve particular positioning of nozzles, possibly with suitable baffles and deflectors to provide suitable combining and direction, but such things are easily provided by the skilled person. Some non-limiting examples include:
(a) two nozzles placed side-by-side, and pointing slightly towards each other, such that their general directions of spray subtend a narrow angle;
(b) the spray direction axis of the fragrance spray nozzle is perpendicular to that of the water spray nozzle, such that the two sprays cross, the quantity and/or force of the water spray being such relative to the fragrance spray that the fragrance spray is entrained.

The mechanism for release of the liquids may be any suitable mechanism. For example, one or both liquids may be under pressure, and depression of valves releases them. Another possibility is to use a spray pump of the type well known to the art, in which the depression of a cap causes liquid to be pumped. This is configured such that the appropriate amounts of liquid are metered for spraying. A further possibility is the use of compressed gas as release agent.

The device is further described with reference to the drawings and the examples, which depict and describe particular embodiments, and which are not intended to be in any way limiting.
Figure 1 is a schematic cross-section of an apparatus used to demonstrate the efficacy of the principle.
Figure 2 is a schematic cross-section of a particular embodiment.
Figure 3 is a perspective schematic view of part of the embodiment of Figure 1.
Figure 4 is a schematic cross-section of a further embodiment.

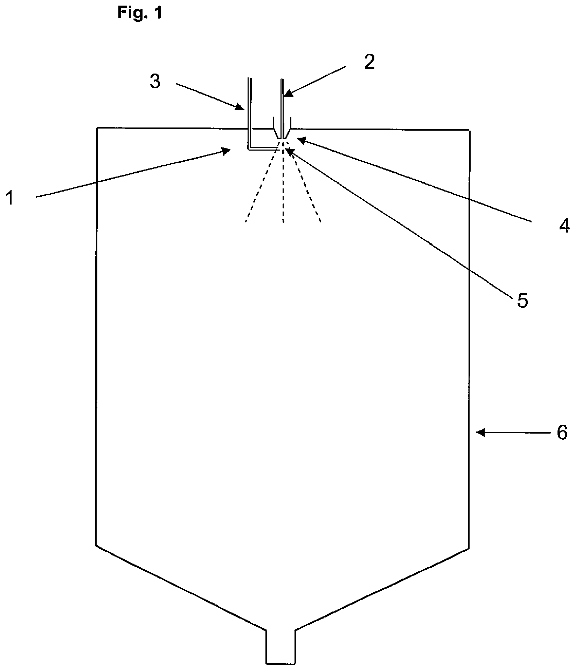

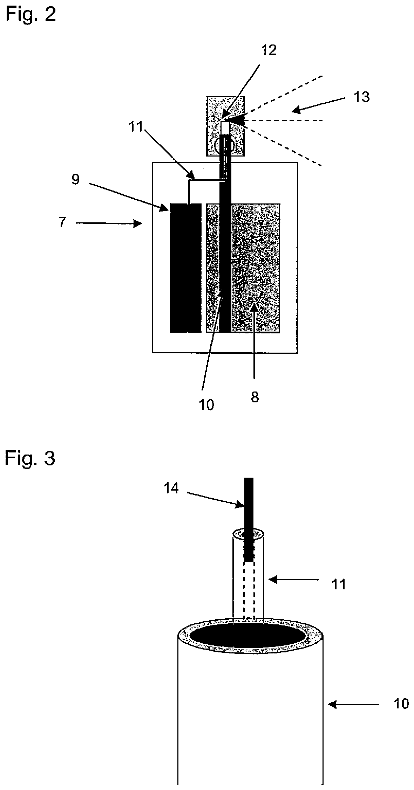

In Figure 1, a spray nozzle, generally indicated as 1 comprises two conduits, one for water 2, the other for fragrance 3. The water conduit 2 leads into a spray nozzle 4. The end 5 of the fragrance conduit 3 is positioned directly in the path of the water spray. Both conduits are fed from reservoirs (not shown) under pressure and the combined spray sprays into a large enclosed volume 6, in this case the collecting chamber of a spray drying apparatus. In Figure 2, a spray container comprises a body portion 7 which houses two reservoirs, a water reservoir 8 and a fragrance reservoir 9. From these reservoirs emerge respectively conduits 10 and 11, leading to a spray cap 12. In this cap, they are mixed and sprayed as a combined water-fragrance spray 13.

Figure 3 shows schematically in more detail that part circled in Figure 2. The water conduit 10 is large in diameter in comparison to the fragrance conduit 11, such that the fragrance conduit can be mounted concentrically within the water conduit, the fragrance conduit having penetrated the wall of the water conduit at a suitable point lower down.

The spray cap 12 is a standard spray cap of the type well known to the art. The cap actuates a pumping mechanism (not shown, but of a type well known to the art), which causes a charge of liquid to be taken from a reservoir and placed in a chamber within the cap, prior to spraying. In this case, two liquids are taken, one from each reservoir, the relative quantities being determined by the relative diameters of the two conduits. Within the cap is also included a downwardly-extending member 14, adapted to fit into and block the fragrance conduit 11, to prevent water or water-fragrance mixture leaking back into the fragrance reservoir and contaminating the fragrance.

In operation, the cap 12 is pumped by applying a series of downward strokes, against the pressure of a helical spring in the cap (not shown) seeking to push the cap away from the spray container. The initial stroke causes the member 14 to withdraw from the fragrance conduit 11 and both liquids to be sucked from their respective reservoirs into the chamber. Succeeding downward strokes cause the chamber to fill and ultimately the mixed liquids to be sprayed from the cap in a fine mist.

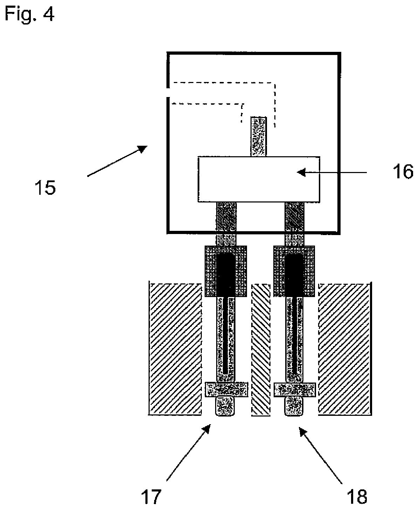

Figure 4 shows a spray cap generally indicated as 15, within which is located a mixing chamber 16 which is fed by two pumps, one 17 for fragrance, the other 18 for water. Depression of the cap 15 causes both pumps to operate and cause liquids to pass from the respective reservoirs to the chamber, prior to spraying. Accurate metering of proportions is ensured by the construction of the pumps, and valves in the pumps prevent any flow back from the chamber 16 into the reservoirs.

### Example 1:

### Demonstration of effective perfume carriage by water aerosol

In this demonstration, the apparatus shown in Figure 1 is used.

99.5 g of deionized water is sprayed into the chamber 4 at room temperature (20 °C), at a feed rate of 9.95 g/min, at an air pressure of 3 bar. At the same time, 0.5 g of a perfume oil concentrate, free of any solvent and comprising ethyl-2 methyl butyrate, prenyl acetate, hexyl acetate, Agrumex™, Jasmacyclene™, beta-ionone, gamma undecalactone and Nectaryl™, is fed to the nozzle at a feed rate of 0.05 g/min, using a flexible tube with an inner diameter of 0.8 mm.

The mixed perfume/water aerosol is collected in the bottom of the spray dryer chamber 6, which is located at a distance of 1 m from the nozzle. The experiment is conducted over a period of 10 minutes and both water and perfume feeds are then stopped. The collected liquid is extracted with cyclohexane and the extract analyzed by capillary gas chromatography in order to determine the amount of perfume present in the liquid. A perfume to water ratio of 0.45 is found, which corresponds to 90% of the nominal perfume to water ratio realized at the level of the nozzle. The composition of the perfume recovered is similar to that of the perfume oil fed in the system, except for the most volatile odorants (e.g. ethyl-2 methyl butyrate, prenyl acetate and citral), the level of which has slightly decreased, due to evaporation.

It is concluded that the water aerosol has effectively carried the perfume through the chamber, with minimal perfume loss and distortion.

### Example 2:

### Working finger sprayer

An apparatus as depicted in Figures 2 and 3 is used, with the perfume fed into water through a capillary tube with a diameter of 0.5 mm, disposed concentrically within a water tube having a larger diameter (2 mm). The opening of the capillary tube is located close to, but still below the opening of the sprayer pump.

The perfume used is a fine fragrance comprising Fixolide™, Galaxolide™, Iso E Super™, Lilial™, dihydro myrcenol, coumarin, cyclohexal, alpha methyl ionone, Radjanol™, Verdantiol™, Hedione™, allyl amyl glycolate, Tricyclal™, aldehyde C12 MNA, dipropylene glycol (9.8%) and diethyl phthalate (8%).

The finger sprayer is then activated until an aerosol is produced. It is found that the aerosol obtained by this action is strongly perfumed and provides a pleasant and strong smell to any substrates, such as skin or clothing, on which the aerosol is applied. The applied aerosol is furthermore judged pleasant and refreshing and does not leave any undesired residues on dark fabrics.

## Claims

1. A method of providing a fine mist of sprayed fragrance, comprising the provision of separate reservoirs (8, 9) of water and fragrance and the conveying of the liquids through separate conduits (10,11) to at least one spray nozzle (12), **characterised in that** the liquids are combined immediately after spraying from the at least one nozzle.

2. A method according to claim 1, in which the spraying takes place through a single nozzle (4), one liquid being sprayed and the other emitted through a conduit (3) located directly in the path of the spray.

3. A method according to claim 1, in which the fragrance conduit (11) is located concentrically within the water conduit (10) for the last parts of their lengths prior to mixing.

4. A method according to claim 1, in which at least one conduit comprises a flow-back-inhibiting valve.

5. A method according to claim 1, in which the spraying takes place through two nozzles, the two sprays being merged.

6. A spray device, comprising separate reservoirs of fragrance and water (8,9), each reservoir having a conduit (10,11) leading therefrom to at least one spray nozzle (12), **characterised in that** the at least one spray nozzle and the conduits are configured such that the two liquids are combined immediately after spraying from the at least one nozzle.

## Patentansprüche

1. Verfahren zur Bereitstellung eines Sprühnebels eines versprühten Duftstoffs, das Bereitstellen separater Wasser- und Duftstoffbehälter (8, 9) und Leiten der Flüssigkeiten durch separate Leitungen (10, 11) zu mindestens einer Sprühdüse (12) umfasst, **dadurch gekennzeichnet, dass** die Flüssigkeiten sofort nach dem Sprühen aus der mindestens einen Düse vereint werden.

2. Verfahren nach Anspruch 1, bei dem das Sprühen durch eine einzige Düse (4) erfolgt, wobei eine Flüssigkeit gesprüht wird und die andere durch eine Leitung (3), die sich direkt in dem Sprühpfad befindet, abgegeben wird.

3. Verfahren nach Anspruch 1, bei dem die Duftstoffleitung (11) konzentrisch in der Wasserleitung (10) für die letzten Teile ihrer Längen vor dem Vermischen positioniert ist.

4. Verfahren nach Anspruch 1, bei dem mindestens eine Leitung ein Rückströmungssperrventil umfasst.

5. Verfahren nach Anspruch 1, bei dem das Sprühen durch zwei Düsen erfolgt, wobei die beiden Sprühmengen zusammengeführt werden.

6. Sprühvorrichtung, die separate Duftstoff- und Wasserbehälter (8, 9) umfasst, wobei jeder Behälter eine Leitung (10, 11) aufweist, die von dort zu mindestens einer Sprühdüse (12) führt, **dadurch gekennzeichnet, dass** die mindestens eine Sprühdüse und die Leitungen so konfiguriert sind, dass die beiden Flüssigkeiten sofort nach dem Sprühen aus der mindestens einen Düse vereint werden.

## Revendications

1. Procédé destiné à fournir un fin nuage de parfum pulvérisé, comprenant la fourniture de réservoirs séparés (8, 9) pour de l'eau et du parfum et le transport des liquides à travers des conduits séparés (10, 11) jusqu'à au moins une buse de pulvérisation (12), **caractérisé en ce que** les liquides sont combinés immédiatement après la pulvérisation à partir de l'au moins une buse.

2. Procédé selon la revendication 1, dans lequel la pulvérisation a lieu à travers une buse (4) unique, un liquide étant pulvérisé et l'autre étant émis à travers un conduit (3) se trouvant directement dans la trajectoire de pulvérisation.

3. Procédé selon la revendication 1, dans lequel le conduit de parfum (11) est situé concentriquement à l'intérieur du conduit d'eau (10) sur les dernières parties de leurs longueurs avant le mélange.

4. Procédé selon la revendication 1, dans lequel au moins un conduit comprend un clapet antireflux.

5. Procédé selon la revendication 1, dans lequel la pulvérisation a lieu à travers deux buses, les deux buses étant regroupées.

6. Dispositif de pulvérisation comprenant des réservoirs séparés de parfum et d'eau (8, 9), chaque réservoir ayant un conduit (10, 11) conduisant depuis celui-ci jusqu'à au moins une buse de pulvérisation (12), **caractérisé en ce que** l'au moins une buse de pulvérisation et les conduits sont configurés de telle sorte que les deux liquides soient combinés immédiatement après la pulvérisation à partir de l'au moins une buse.
